# EUROPEAN PATENT APPLICATION

(11) **EP 4 245 333 A1**
(43) Date of publication of application: **20.09.2023**
(21) Application number: 20962408.9
(22) Date of filing: 18.11.2020
(51) Int. Cl.: A61M 5/158, A61M 25/02, A61M 25/06

(54) **INDWELLING NEEDLE**

(71) Applicant: TERUMO Kabushiki Kaisha, Tokyo 151-0072 (JP)
(72) Inventor: FUJIEDA, Ryohei, Laguna, 4024 (PH); CABANTING, Jelly, Laguna, 4024 (PH); MUNSOD, Anna, Laguna, 4024 (PH); AKAIKE, Toshiyuki, Laguna, 4024 (PH)
(74) Representative: Casalonga
(86) International application number: PCT/JP2020/042989
(87) International publication number: WO 2022/107244

(57) **Abstract**

An indwelling needle (10) includes a catheter (12), a catheter hub (14), and an inner needle (18). The catheter hub (14) includes a hub body (30) and a wing (40) protruding from a side portion of the hub body (30). The wing (40) includes a soft portion (44) softer than the catheter hub (14) and a hard portion (42) harder than the soft portion (44). The hard portion (42) has a front frame (54) which is located on a distal side of the wing (40), is connected to the hub body (30), and extends in a direction away from the hub body (30). The soft portion (44) covers at least a surface of the front frame (54) opposing a body surface X of a living body.

## Description

### Technical Field

The present invention relates to an indwelling needle that is inserted into a body of a living body and indwell on a body surface.

### Background Art

An indwelling needle includes a catheter, a catheter hub that holds a proximal end of the catheter, and an inner needle inserted into the catheter. A user of the indwelling needle inserts the catheter and the inner needle into a body such as a vein of a patient (living body), and then detaches the inner needle from the catheter, thereby placing the catheter to indwell inside a blood vessel and placing the catheter hub to indwell on a body surface. In an indwelling state, the indwelling needle introduces a fluid such as a medicinal liquid into the blood vessel through a flow channel in the catheter.

In addition, as disclosed in JP 2005-512610 W, an indwelling needle includes a wing in order to improve operability when a patient is punctured with a catheter and an inner needle and facilitate indwelling of a catheter hub on a body surface. Furthermore, the indwelling needle includes a reinforcing member in the wing to increase rigidity of the wing.

### Summary of Invention

Meanwhile, as a way of using this type of indwelling needle, there is a method in which a user grips the indwelling needle by hooking a finger on a front edge of the wing in order to accurately perform puncture with the catheter. In a case where the indwelling needle is gripped by such a method, the wing is required to have hardness not to be bent. In the indwelling needle disclosed in JP 2005-512610 W, the reinforcing member is configured separately from the catheter hub, and thus, the wing is likely to be bent at the time of gripping when the above-described gripping method is used. Furthermore, the wing is required to have softness (a property opposite to the hardness described above) in order to ensure comfort of the patient during indwelling of the indwelling needle on the body surface.

The present invention relates to a technique of the above-described indwelling needle, and an object thereof is to provide an indwelling needle capable of enhancing comfort in an indwelling state while enhancing operability at the time of puncture.

In order to achieve the above object, an aspect of the present invention an indwelling needle including: a catheter; a catheter hub fixed to a proximal end of the catheter; and an inner needle that is detachably inserted into the catheter and has a needle tip protruding from a distal end of the catheter. The catheter hub includes a hub body and a wing protruding from a side portion of the hub body. The wing includes a soft portion softer than the catheter hub and a hard portion connected to the hub body and harder than the soft portion. A part of the wing that abuts on a body surface is configured by the soft portion. The hard portion includes a front frame located on a distal side of the wing. The front frame is connected to the hub body without interposing the soft portion, and extends in a direction apart from the hub body.

The above-described indwelling needle can enhance the comfort in the indwelling state while enhancing the operability at the time of puncture.

### Brief Description of Drawings

Fig. 1 is a perspective view illustrating an overall configuration of an indwelling needle according to an embodiment of the present invention.
Fig. 2 is an exploded perspective view illustrating the overall configuration of the indwelling needle.
Fig. 3 is a side cross-sectional view illustrating a part of the indwelling needle on a proximal side.
Fig. 4A is a plan view illustrating a hub body and wings. Fig. 4B is a side view illustrating the hub body and the wings. Fig. 4C is a perspective view of the hub body and the wings as viewed obliquely from below.
Fig. 5A is a plan view illustrating a soft portion. Fig. 5B is a front view of the soft portion. Fig. 5C is a perspective view of the soft portion as viewed obliquely from below.
Fig. 6 is a cross-sectional view taken along line VI-VI of Fig. 3.
Fig. 7 is a perspective view illustrating an operation of the indwelling needle at the time of puncture.
Fig. 8A is a side view illustrating an indwelling state of the indwelling needle. Fig. 8B is a partial side cross-sectional view illustrating an example of the indwelling state of the wings.
Fig. 9A is a plan view illustrating wings according to a first modification. Fig. 9B is a plan view illustrating wings according to a second modification. Fig. 9C is a plan view illustrating wings according to a third modification. Fig. 9D is a plan view illustrating wings according to a fourth modification.
Fig. 10A is a perspective view illustrating wings according to a fifth modification. Fig. 10B is a cross-sectional view taken along line XB-XB of Fig. 10A.

### Description of Embodiments

Hereinafter, preferred embodiments of the present invention will be described in detail with reference to the accompanying drawings.

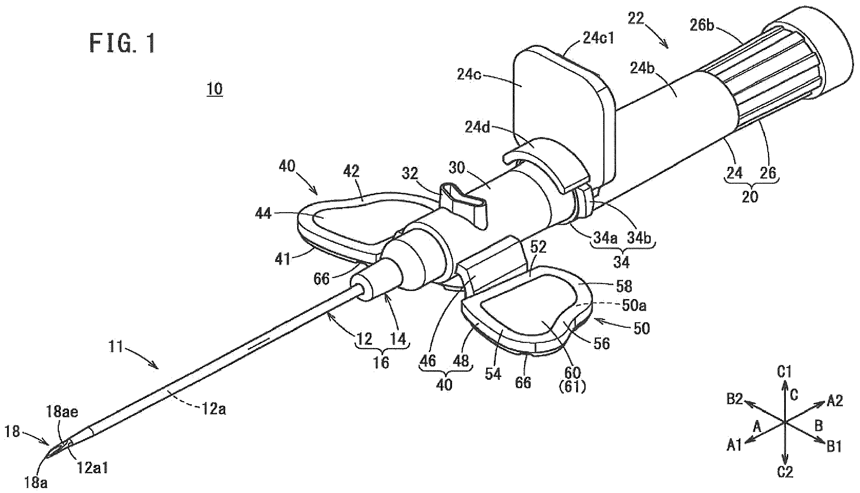

An indwelling needle 10 according to an embodiment of the present invention has a catheter 12 that is inserted and indwell in a patient's body (living body) as illustrated in Fig. 1, and is used to construct an inlet/outlet for a liquid (a medicinal liquid and blood) during an infusion, a blood transfusion, blood sampling, or the like. The catheter 12 according to the present embodiment is a venous catheter inserted into a peripheral vein. Incidentally, the catheter 12 may be a catheter longer than the peripheral venous catheter (for example, a central venous catheter, a PICC, a mid-line catheter, and the like). In addition, the catheter 12 is not limited to the venous catheter, and may be an arterial catheter such as a peripheral arterial catheter.

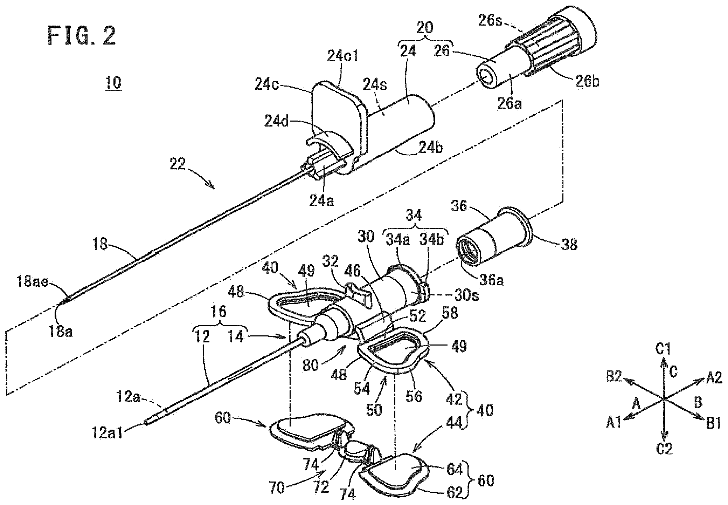

As illustrated in Figs. 1 and 2, the indwelling needle 10 includes a catheter indwelling body 16 including the above-described catheter 12 extending in a longitudinal direction (an arrow A direction) and a catheter hub 14 fixed to a proximal side (an arrow A2 side) of the catheter 12. In addition, the indwelling needle 10 includes an inner needle operation body 22 including an inner needle 18 extending in an axial direction (the arrow A direction) and a needle hub 20 fixed to the proximal side of the inner needle 18.

In the indwelling needle 10, a multi-structure needle 11 in which the inner needle 18 is inserted into the catheter 12 is formed as the inner needle operation body 22 is assembled to a proximal end of the catheter indwelling body 16 in an initial state before use (in a form of being provided as a product). A needle tip 18a of the inner needle 18 protrudes from a distal end of the catheter 12 toward a distal side (an arrow A1 side) in the initial state.

The inner needle 18 is formed in a hollow tube or a solid rod shape having rigidity capable of puncturing the skin of the living body. The needle tip 18a is formed to have a sharp blade surface 18ae at a distal end of the inner needle 18. The blade surface 18ae faces the distal side and the upper side (lower side). Examples of a constituent material of the inner needle 18 include a metal material such as stainless steel, aluminum or an aluminum alloy, and titanium or a titanium alloy, a hard resin, ceramics, and the like. The inner needle 18 is firmly fixed to the needle hub 20 by an appropriate fixing means such as fusion, adhesion, and insert molding.

The needle hub 20 includes a connection member 24 connected to a proximal end of the catheter hub 14 and a proximal member 26 connected to a proximal end of the connection member 24. The connection member 24 is formed in a cylindrical shape to be thicker than the catheter hub 14. The connection member 24 includes a holding portion 24a that holds a proximal end of the inner needle 18 on the distal side, and a tube portion 24b extending from the holding portion 24a in a proximal direction. In addition, the connection member 24 has a protruding piece 24c protruding in a direction orthogonal to an axial center of the connection member 24 at a distal end of the tube portion 24b, and has a cover 24d protruding from the protruding piece 24c toward the distal side.

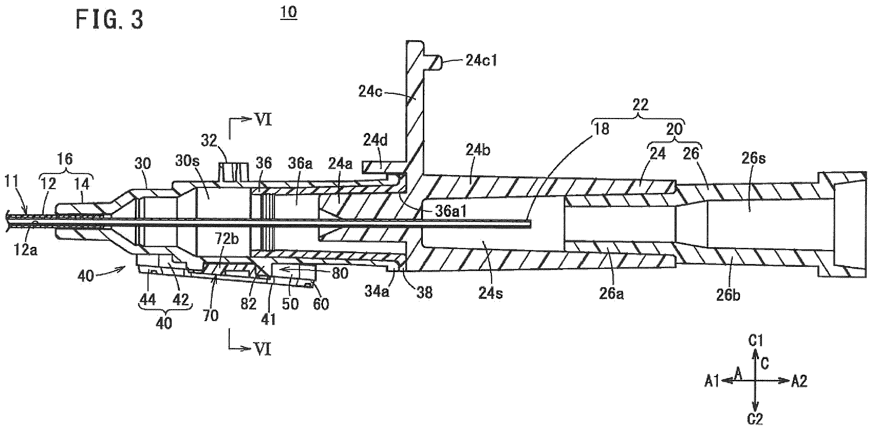

As illustrated in Figs. 2 and 3, the holding portion 24a includes a central tube portion (not illustrated) that fixes the inner needle 18, and a plurality of ridges (not illustrated) that protrude radially outward from an outer peripheral surface of the central tube portion and extend along the axial direction of the connection member 24. The plurality of ridges are frictionally fitted to an inner wall of the catheter hub 14 (an inner member 36 to be described later).

The tube portion 24b is formed in a cylindrical shape having a larger diameter than the holding portion 24a, and is exposed on the proximal side with respect to the catheter hub 14. The tube portion 24b has a space portion 24s, which is blocked on the distal side by the holding portion 24a, therein. The space portion 24s communicates with a proximal open portion of the tube portion 24b, and a proximal end opening of the inner needle 18 fixed to the holding portion 24a is disposed therein. In addition, the proximal member 26 is inserted into the proximal side of the space portion 24s through the proximal open portion. For example, the tube portion 24b is formed to be transparent or translucent, so that blood flowing out from the proximal end opening of the inner needle 18 can be visually recognized.

The protruding piece 24c is formed in a plate shape protruding high toward the upper side of the indwelling needle 10 (an arrow C1 side: a direction that the blade surface 18ae faces) . A length in a width direction (an arrow B direction) and a length in a vertical direction (an arrow C direction) of the protruding piece 24c are set to be equal to or larger than the diameter of the tube portion 24b. The protruding piece 24c is formed to be hard with an appropriate thickness along the axial direction of the connection member 24. The protruding piece 24c constitutes a part that abuts against a thumb or the like when a user performs puncture with the indwelling needle 10. An upper end of the protruding piece 24c is provided with a projection 24c1 protruding to the proximal side in order to be easily caught by the thumb or the like.

The cover 24d is formed in an arc shape and partially covers an upper portion of the catheter hub 14 on the proximal side. The cover 24d restricts the connection member 24 from rotating relative to the catheter hub 14 in cooperation with a pair of lateral protrusions 34b of the catheter hub 14 to be described later. Therefore, the relative rotation of the inner needle 18 with respect to the catheter 12 is restricted in the indwelling needle 10 in the initial state.

The proximal member 26 is formed in a cylindrical shape, and includes a distal connecting portion 26a that is inserted to be tapered and fitted to the space portion 24s of the connection member 24. A plurality of ribs extending along the axial direction of the proximal member 26 are provided on an outer peripheral surface of a body portion 26b on the proximal side with respect to the distal connecting portion 26a. A space portion 26s in the proximal member 26 extends inside the distal connecting portion 26a and the body portion 26b, and communicates with the space portion 24s of the connection member 24 in the initial state. For example, a filter (not illustrated) is provided in the space portion 26s. The filter is configured to be capable of deflating air in the space portions 24s and 26s while preventing passage of blood flowing out from the proximal end opening of the inner needle 18.

As illustrated in Figs. 1 to 3, the catheter 12 is configured as a hollow flexible tube and has a lumen 12a extending along the axial direction of the catheter 12 therein. The lumen 12a communicates with a distal opening 12a1 of the catheter 12 and a proximal opening (not illustrated) of the catheter 12. A proximal end of the catheter 12 is inserted inside the catheter hub 14 and fixed to the catheter hub 14 by an appropriate fixing means such as caulking, fusion, and adhesion.

A material forming the catheter 12 is not particularly limited, but a transparent soft resin material may be applied. Examples of a constituent material of the catheter 12 include a fluorine-based resin such as polytetrafluoroethylene (PTFE), an ethylene-tetrafluoroethylene copolymer (ETFE), and a perfluoroalkoxy fluorine resin (PFA), an olefin-based resin such as polyethylene and polypropylene or a mixture thereof, polyurethane, polyester, polyamide, a polyether nylon resin, a mixture of the olefin-based resin and an ethylene-vinyl acetate copolymer, and the like.

When the catheter 12 is inserted into a blood vessel of the patient, the catheter hub 14 is exposed on and pasted, with a tape or the like, to a body surface (skin) of the patient to indwell together with the catheter 12. The catheter hub 14 according to the present embodiment includes a hub body 30 to which the catheter 12 is directly fixed, the inner member 36 mounted inside the hub body 30 from a proximal end of the hub body 30, and a pair of wings 40 protruding from a side portion of the hub body 30.

The hub body 30 is formed in a cylindrical shape tapered stepwise toward the distal side, and includes a hollow portion 30s extending in the axial direction of the hub body 30 therein. The catheter 12 is inserted into a distal end of the hollow portion 30s, and the catheter 12 is fixed to an inner peripheral surface of a distal end of the hub body 30. In addition, the hollow portion 30s communicates with the proximal opening of the catheter 12.

A tab 32 that allows the user to push out the catheter hub 14 is provided on the upper side of an outer peripheral surface of the hub body 30. A flange 34 protruding from the outer peripheral surface of the hub body 30 is formed at the proximal end of the hub body 30. The flange 34 has an annular portion 34a protruding low radially outward and extending in a circumferential direction of the hub body 30, and the pair of lateral protrusions 34b protruding slightly high from a side surface (at positions shifted by ±90° with respect to the tab 32) of the annular portion 34a in the width direction. In the indwelling needle 10, the cover 24d of the needle hub 20 is disposed on the annular portion 34a between the pair of lateral protrusions 34b.

The inner member 36 is inserted into the hollow portion 30s from the proximal end of the hub body 30 and is tapered and fitted to an inner peripheral surface of the hub body 30. The inner member 36 functions as a restricting portion that prevents a member (not illustrated) provided in the hollow portion 30s from dropping out. Examples of the member in the hollow portion 30s include a hemostasis valve, a plug for opening the hemostasis valve, a filter for preventing outflow of blood, and the like.

A through-hole 36a penetrating through the inner member 36 in the axial direction and communicating with the hollow portion 30s is provided inside the inner member 36. The through-hole 36a communicates with a proximal open portion 36a1 provided at the proximal end of the inner member 36. The proximal open portion 36a1 constitutes the proximal end opening of the catheter hub 14. An annular protrusion 38 protruding radially outward is provided at the proximal end of the inner member 36, and the protrusion 38 is disposed to overlap a proximal end of the annular portion 34a.

A constituent material of the above catheter hub 14 is not particularly limited, but a thermoplastic resin such as polypropylene, polycarbonate, polyamide, polysulfone, polyarylate, or a methyl methacrylate-butadiene-styrene copolymer can be applied. The hub body 30 and the inner member 36 may be made of the same material or may be made of different materials.

As illustrated in Figs. 1 and 2, the pair of wings 40 of the catheter hub 14 is provided to improve operability when the user punctures the patient with the multi-structure needle 11, facilitate the indwelling of the catheter hub 14 on the body surface, and improve durability. The pair of wings 40 is connected to the outer peripheral surface in the width direction which is a side portion of the hub body 30, and extends outward in the width direction of the hub body 30 (direction apart from the hub body 30). Incidentally, in the following description of the pair of wings 40, the wing 40 on the left side (an arrow Bl side) as viewed from the user at the time of puncture with the indwelling needle 10 will be described as a representative, and the description of the wing 40 on the right side (an arrow B2 side) symmetrical thereto will be omitted.

The wing 40 includes two types of portions, that is, a hard portion 42 and a soft portion 44. The hard portion 42 is made of the same material as the hub body 30, and is integrally molded with the hub body 30. The hard portion 42 has hardness (rigidity: a high elastic modulus) not to be elastically deformed by a force applied from the user at the time of puncture. The soft portion 44 is a member made of a material softer than the catheter hub 14 and the hard portion 42. The indwelling needle 10 is provided in a form in which the soft portion 44 is mounted to the hard portion 42 to be integrally supported.

More specifically, the wing 40 has a root portion 46 which is a part connected to the outer peripheral surface of the hub body 30, and a plate-shaped wing portion 48 which is connected to a protruding end 46a of the root portion 46 and extends in the arrow B direction.

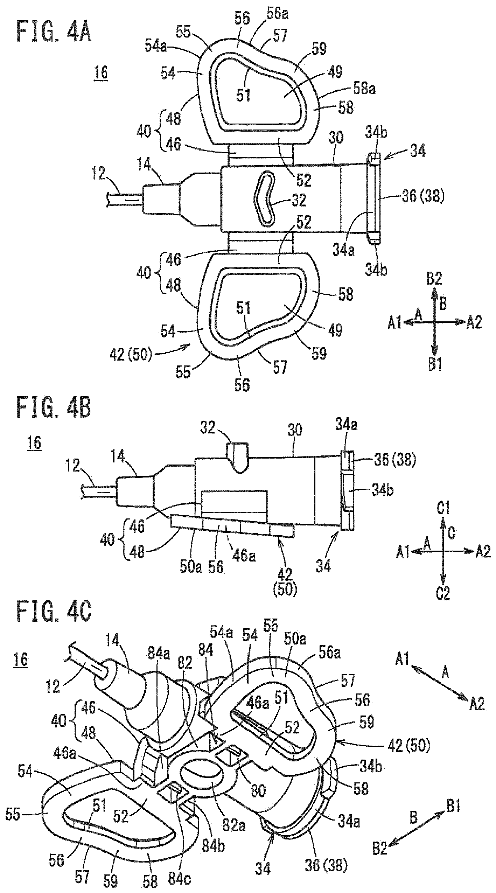

As illustrated in Figs. 2 and 4A to 4C, the root portion 46 constitutes a part of the hard portion 42. The root portion 46 protrudes short to the left side from the outer peripheral surface on the left side of the hub body 30, and then, extends obliquely toward the left side and the lower side. The root portion 46 is provided at a substantially central portion in the axial direction of the hub body 30 and has a predetermined length (about 1/3 of the entire length of the hub body 30) over the axial direction.

The root portion 46 has a sufficient thickness and is configured not to be elastically deformable. In addition, the root portion 46 is connected to a support reinforcing portion 80 formed below the root portion 46 and the hub body 30.

The protruding end 46a of the root portion 46 protrudes to the lower side with respect to the outer peripheral surface of the hub body 30. In a side view of the catheter hub 14, the distal side of the protruding end 46a is located near the hub body 30 (at an upper position), and the proximal side of the protruding end 46a is located at a lower position farther away from the hub body 30 than the distal side of the protruding end 46a. Therefore, the protruding end 46a is inclined so as to approach an axial center of the hub body 30 toward the distal side of the hub body 30.

Returning to Fig. 1, the wing portion 48 connected to the root portion 46 has a frame 50 that is configured by the hard portion 42 and serves as a skeleton of the wing portion 48, and a planar portion 60 configured by the soft portion 44. That is, the wing portion 48 has a plate shape as a whole as the planar portion 60 is disposed in a window portion 49 formed on the inner side of the frame 50. In addition, the planar portion 60 is attached to the frame 50 so as to cover a back surface 50a (an opposing surface that opposes the body surface) of the frame 50.

As illustrated in Figs. 4A to 4C, the frame 50 is continuous with the protruding end 46a of the root portion 46, and protrudes outward in the width direction from the protruding end 46a. The frame 50 is formed in a prismatic shape and has a sufficient thickness in the arrow C direction. As a result, the frame 50 has hardness (rigidity: a high elastic modulus) not to be elastically deformed even by the force from the user toward the proximal side at the time of puncture. Incidentally, since the window portion 49 is provided on the inner side of the frame 50, the wing 40 (frame 50) can be elastically deformed in the vertical direction (a direction orthogonal to the axial direction of the hub body 30 and a direction orthogonal to an extending direction of a front frame 54 to be described later) as a whole.

The frame 50 has a connecting frame 52 extending along the protruding end 46a of the root portion 46 on the inner side of the wing portion 48. The connecting frame 52 protrudes longer to the distal side and the proximal side than the root portion 46. Since the connecting frame 52 extends along the inclination of the protruding end 46a of the root portion 46, the frame 50 is inclined so as to approach the axial center of the hub body 30 toward the distal side of the hub body 30 as a whole.

In addition, the frame 50 includes the front frame 54 extending on the distal side of the wing portion 48, a side frame 56 extending on the outer side of the wing portion 48, and a rear frame 58 extending on the proximal side of the wing portion 48. The front frame 54 is connected to a distal portion of the connecting frame 52. The rear frame 58 is connected to a proximal portion of the connecting frame 52. In a plan view of the wing 40, a width of the connecting frame 52, a width of the front frame 54, a width of the side frame 56, and a width of the rear frame 58 are set to be the same with each other. The "width" of each of the connecting frame 52, the front frame 54, the side frame 56, and the rear frame 58 is a dimension in a direction orthogonal to the extending direction of each frames. That is, the frame 50 extends along an outer peripheral portion of the wing portion 48 with a constant width. The width and thickness of the frame 50 are set in a range of about 1 mm to 10 mm, for example.

The front frame 54 extends outward in the width direction to be long from the connecting frame 52 and extends somewhat toward the distal side from the distal end of the connecting frame 52. In the present embodiment, a distal end side 54a of the front frame 54 is located on the most distal side in the wing 40. In a side view of the hub body 30, when the hub body 30 is divided into three equal parts in the axial direction, the distal end side 54a is provided to be located at a distal part (on the distal side) among the three equal parts. An end of the front frame 54 on the outer side in the width direction is smoothly continuous with an end of the side frame 56 on the distal side via a curved portion 55 having a large radius of curvature.

The side frame 56 is connected to the front frame 54 and the rear frame 58, and extends outward in the width direction of the wing 40. The side frame 56 is inclined inward in the width direction (toward the catheter hub 14) from the distal side to the proximal side. A constricted portion 57 that is gently constricted inward is provided in a substantially intermediate portion of the side frame 56 in the arrow A direction. In the present embodiment, the lateral side 56a of the side frame 56 is located on the outermost side in the wing 40. An end of the side frame 56 on the proximal side is smoothly continuous with an end of the rear frame 58 on the outer side in the width direction via the bent portion 59 having a large radius of curvature.

The rear frame 58 extends short between the connecting frame 52 and the side frame 56, and is formed in an arc shape bulging somewhat toward the proximal side from the proximal end of the connecting frame 52. An extending length of the rear frame 58 is shorter than an extending length of the front frame 54. In the present embodiment, a proximal end side 58a of the rear frame 58 is located on the most proximal side in the wing 40.

The frame 50 is formed in an annular shape by the connecting frame 52, the front frame 54, the side frame 56, and the rear frame 58. An inner peripheral surface of the annular frame 50 has an engagement protrusion 51 for mounting of the planar portion 60 of the soft portion 44. The engagement protrusion 51 is provided to be continuous with each of the frames and is formed to be thinner than the thickness of each of the frames. The engagement protrusion 51 makes a circuit with the same protruding length inside the connecting frame 52, the front frame 54, the side frame 56, and the rear frame 58. On the back surface 50a of the frame 50, the connecting frame 52, the front frame 54, the side frame 56, the rear frame 58, and the engagement protrusion 51 are flush with each other. Therefore, the back surface 50a of the frame 50 is formed in a flat shape.

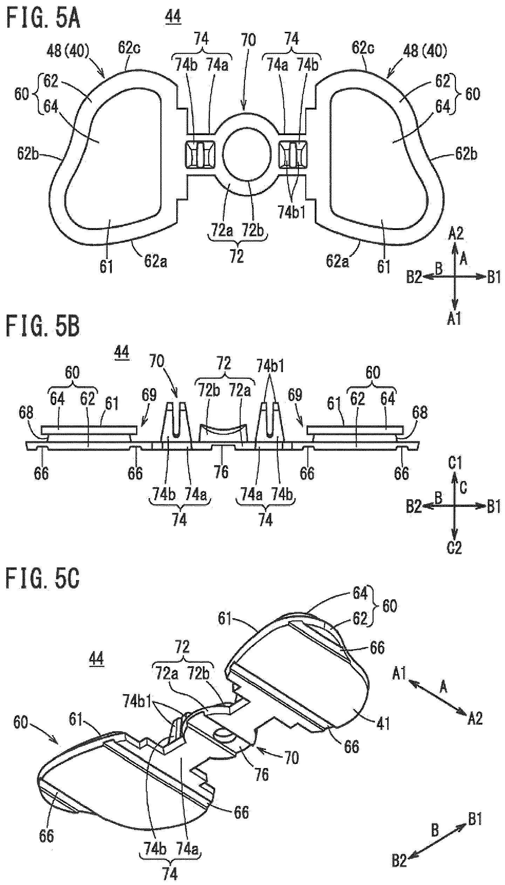

As illustrated in Figs. 5A to 5C, the planar portion 60 of the soft portion 44 has a base portion 62 disposed on the back surface 50a side of the frame 50 and a thick portion 64 integrally molded with the base portion 62 on an upper surface of the base portion 62. The thick portion 64 is a part to be inserted into the window portion 49 surrounded by the frame 50.

The base portion 62 substantially matches a shape of the frame 50 (the connecting frame 52, the front frame 54, the side frame 56, and the rear frame 58) in the plan view. That is, the base portion 62 has a distal end side 62a, a lateral side 62b, and a proximal end side 62c respectively having the same shapes as the distal end side 54a, the lateral side 56a, and the proximal end side 58a of the frame 50. The base portion 62 has a thickness thinner than the thickness of the frame 50. An upper surface of the base portion 62 on which the thick portion 64 is not provided is formed as a flat surface and serves as a portion that is in close contact with the back surface 50a of the frame 50.

A lower surface of the base portion 62 constitutes a lower surface 41 of the wing 40. One or more (in Fig. 5B, two) grooves 66 are provided in the lower surface of the base portion 62. Each of the grooves 66 extends on the lower surface of the base portion 62 along the arrow A direction, opens to the distal side at the distal end side 62a of the base portion 62, and opens to the proximal side at the proximal end side 62c. The lower surface of the base portion 62 other than a site where the groove 66 is formed as a flat surface and is continuous in an R shape with an edge of the groove 66. Incidentally, an extending direction of the groove 66 is not limited to the arrow A direction, and may be another direction.

The thick portion 64 is formed to have the same thickness as the thickness of the frame 50 on the base portion 62. An upper surface of the thick portion 64 is configured as a flat surface. In a state where the planar portion 60 is mounted to the frame 50, the upper surface of the thick portion 64 is flush with an upper surface of the frame 50 (see Fig. 1). In addition, an outer peripheral surface of the thick portion 64 matches the shape of the inner peripheral surface of the frame 50 in the plan view.

An engagement recess 68 recessed inward of the thick portion 64 is formed on the outer peripheral surface of the thick portion 64. The engagement recess 68 makes a circuit of the entire outer peripheral surface of the thick portion 64 in the circumferential direction at a position adjacent to the base portion 62. The engagement recess 68 of the planar portion 60 and the engagement protrusion 51 of the frame 50 constitute an engagement structure 69 for mounting the planar portion 60 to the frame 50.

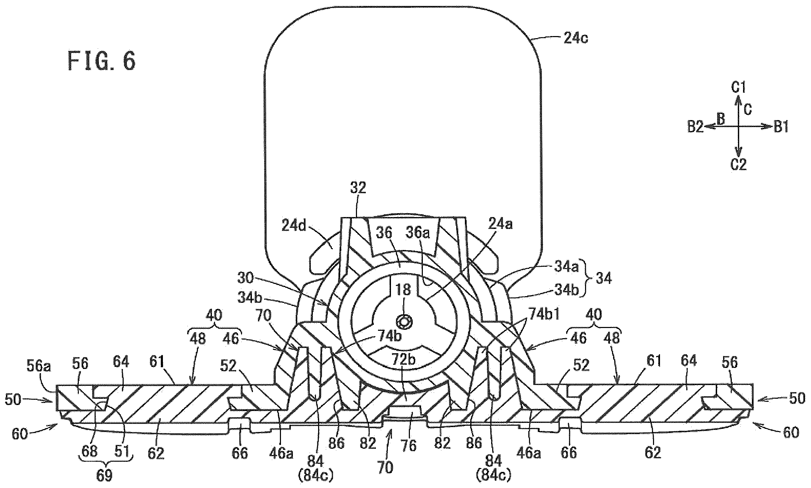

That is, as illustrated in Fig. 6, the engagement structure 69 prevents the thick portion 64 from coming out of the frame 50 as the engagement protrusion 51 is inserted into the engagement recess 68 to be hooked with each other. Since the engagement recess 68 and the engagement protrusion 51 engage with each other over the entire circumference of the inner peripheral surface of the frame 50, the planar portion 60 is restricted from moving in a plane direction orthogonal to a thickness direction of the frame 50, and is firmly integrated with the frame 50.

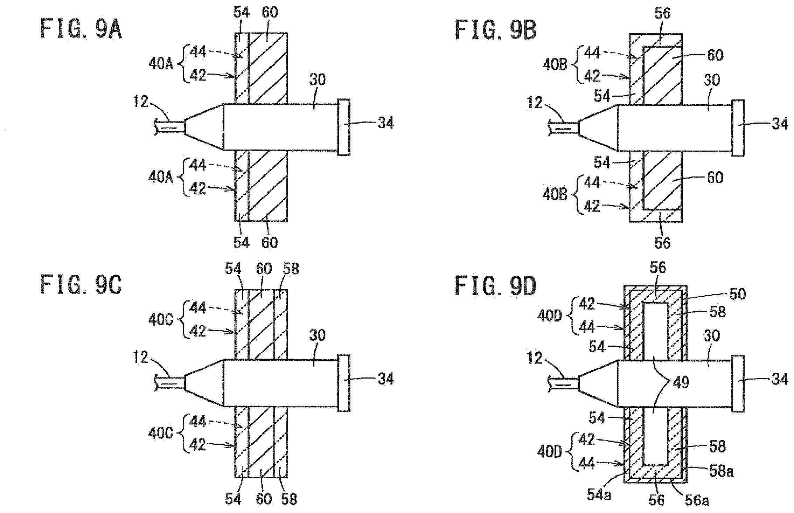

In addition, the planar portion 60 constitutes a visual recognition portion 61 in which the body surface of the patient can be visually recognized through the base portion 62 and the thick portion 64 inside the wing portion 48. The visual recognition portion 61 is achieved by the soft portion 44 (the base portion 62 and the thick portion 64) having transparency or translucency. Incidentally, the visual recognition portion 61 may be configured using, for example, one or more holes (not illustrated) penetrating through the planar portion 60. In addition, the wing 40 may be configured such that the soft portion 44 is provided on the back surface 50a of the frame 50 and the soft portion 44 is not provided on the inner side of the frame 50, so that the window portion 49 itself may be used as the visual recognition portion 61 (see also Fig. 9D).

As illustrated in Figs. 5A to 5C, the soft portion 44 is configured as one member in which the planar portions 60 provided in a pair corresponding to the pair of wings 40 are connected. Therefore, the soft portion 44 includes a connecting structure 70 that connects the planar portions 60 and is provided between the pair of planar portions 60. The connecting structure 70 includes a hub opposing portion 72 provided at a central portion in the width direction and a pair of connecting arms 74 extending between the hub opposing portion 72 and each of the planar portions 60.

The hub opposing portion 72 is provided so as to be located below the hub body 30. The hub opposing portion 72 includes a disk portion 72a, which has the same thickness as the thickness of the base portion 62 and is formed in a circular shape in the plan view, and an upper protruding portion 72b protruding from an upper surface of the disk portion 72a. One or more (one in Fig. 5B) grooves 76 extending in the axial direction of the hub body 30 are provided on a lower surface of the disk portion 72a. The groove 76 opens at outer edges on the distal side and the proximal side of the disk portion 72a.

The upper protruding portion 72b is inserted into the support reinforcing portion 80 provided to be continuous with the hub body 30. An upper end surface of the upper protruding portion 72b is formed as a curved surface that can come into close contact with the outer peripheral surface of the hub body 30.

The pair of connecting arms 74 extends from the hub opposing portion 72 by a length of the root portion 46 in the width direction. Each of the connecting arms 74 includes a rectangular portion 74a, which has the same thickness as the base portion 62 and is formed in a quadrangular shape in the plan view, and an engagement body 74b protruding from an upper surface of the rectangular portion 74a.

The engagement body 74b has a pair of engagement pieces 74bl bifurcated from a site provided to be continuous with the rectangular portion 74a and protruding upward. The pair of engagement pieces 74b1 is formed upward to be higher than the upper protruding portion 72b. In addition, outer side surfaces of the pair of engagement pieces 74b1 are inclined in directions approaching each other toward the upper side.

The material constituting the soft portion 44 is not particularly limited, but a thermosetting elastomer or a thermoplastic elastomer, for example, is preferably applied. In addition, the planar portion 60 of the soft portion 44 is configured to have transparency or translucency in order to enable the visual recognition of the body surface as described above. Therefore, as the material constituting the soft portion 44, an acrylic elastomer, a styrene elastomer, or a polyolefin elastomer, which is a transparent or translucent material among elastomers, may be adopted.

Meanwhile, the support reinforcing portion 80 of the hub body 30 is integrally molded with the hub body 30, and supports the above-described connecting structure 70 to reinforce the mounting of the soft portion 44 as illustrated in Figs. 4A to 4C. The support reinforcing portion 80 includes a support tube portion 82 protruding downward from the outer peripheral surface of the hub body 30, and a pair of connecting wall portions 84 connected to the inner side of the root portion 46 and the wing portion 48 on both side surfaces of the support tube portion 82 in the width direction.

The support tube portion 82 protrudes short so as to be at the same position as the protruding end 46a of the root portion 46 and the back surface 50a of the frame 50. A hole portion 82a that exposes the outer peripheral surface of the hub body 30 is provided on the inner side of the support tube portion 82. As the upper protruding portion 72b is inserted into the hole portion 82a, an inner peripheral surface of the support tube portion 82 is frictionally fitted to an outer peripheral surface of the upper protruding portion 72b. As a result, the support tube portion 82 firmly supports the hub opposing portion 72 and promotes the integration of the hard portion 42 and the soft portion 44.

Each of the connecting wall portions 84 has a distal lateral wall 84a and a proximal lateral wall 84b extending in the width direction at a side portion of the support tube portion 82, and one partition wall 84c provided between the distal lateral wall 84a and the proximal lateral wall 84b. The distal lateral wall 84a and the proximal lateral wall 84b are connected to the support tube portion 82 and the root portion 46 to form an insertion hole 86 into which the engagement body 74b of the soft portion 44 can be inserted. The partition wall 84c partitions the insertion hole 86 into two parts along the width direction, and divides and holds the pair of engagement pieces 74b1 inserted between a wall surface of the support tube portion 82 and a wall surface of the root portion 46 as illustrated in Fig. 6. As a result, the connecting wall portion 84 firmly supports the connecting arm 74 and promotes the integration of the hard portion 42 and the soft portion 44.

The indwelling needle 10 according to the present embodiment is basically configured as described above, and operations thereof will be described hereinafter.

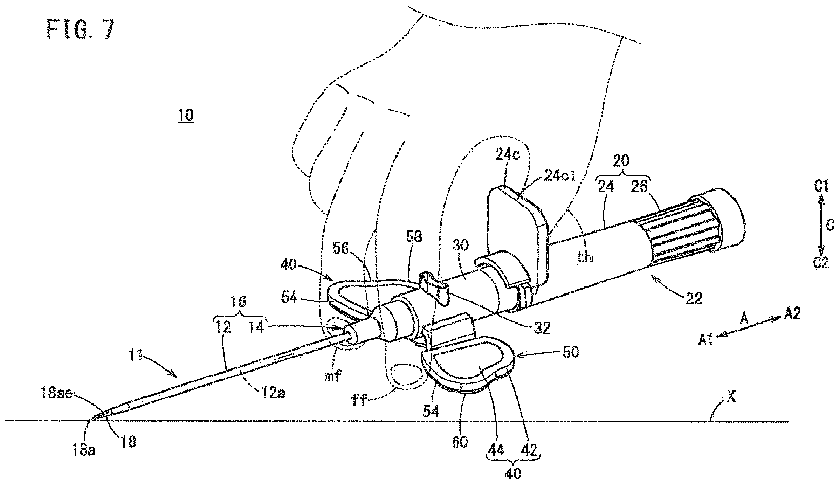

As described above, the indwelling needle 10 is used at the time of constructing the inlet/outlet for the infusion, the blood transfusion, the blood sampling, and the like with respect to the patient. At this time, the user such as a doctor or a nurse grips the indwelling needle 10 in the initial state in a gripping form as illustrated in Fig. 7, and punctures a body surface X of the patient with the multi-structure needle 11.

Specifically, the user takes a posture in which two fingers (an index finger ff and a middle finger mf) straddle the hub body 30 above the hub body 30 of the catheter hub 14. Then, the user puts a thumb th on the upper end (projection 24c1) of the protruding piece 24c while hooking tips of the two fingers on the pair of wings 40, respectively. At this time, the two fingers are in contact with the distal side (the front frame 54) of each of the wings 40. Then, the indwelling needle 10 can be stably gripped by applying a force toward the proximal side with respect to the pair of wings 40 using the two fingers and a force toward the distal side with respect to the protruding piece 24c using the thumb th.

The indwelling needle 10 includes the hard portion 42 (front frame 54) on the distal side of each of the pair of wings 40, and the front frame 54 is connected to the hub body 30 without interposing the soft portion 44 and extends in the direction of being separated from the hub body 30. The front frame 54 is not deformed toward the proximal side even if the force toward the proximal side is applied from the user's finger. Therefore, the pair of wings 40 can stably transmit the gripping force of the user to the hub body 30 through the front frame 54. In particular, the distal end side 54a of the front frame 54 is a part that directly receives the force of the finger at the distal end of the wing 40, so that the force of the user can be easily applied.

The needle tip 18a of the multi-structure needle 11 reaches the inside of the blood vessel by the puncture operation of the user. The user inserts the catheter 12 into the blood vessel by advancing the catheter 12 relative to the inner needle 18 while maintaining the puncture. At this time, the user can easily advance the catheter 12 by sandwiching and gripping the needle hub 20 (or one of the pair of wings 40 and the protruding piece 24c) with the thumb th and the middle finger mf and pushing out the tab 32 of the catheter hub 14 with the index finger ff.

After inserting the catheter 12 into the blood vessel, the user retracts the inner needle operation body 22 relative to the catheter indwelling body 16 and removes the inner needle 18 from the catheter 12 and the catheter hub 14. As a result, the catheter hub 14 remains on the body surface X of the patient. A connector of a medical device (not illustrated) is connected to the catheter hub 14 from a proximal portion. As a result, the catheter indwelling body 16 (indwelling needle 10) performs a treatment such as administration of a medicinal liquid or blood to the patient and blood sampling from the patient.

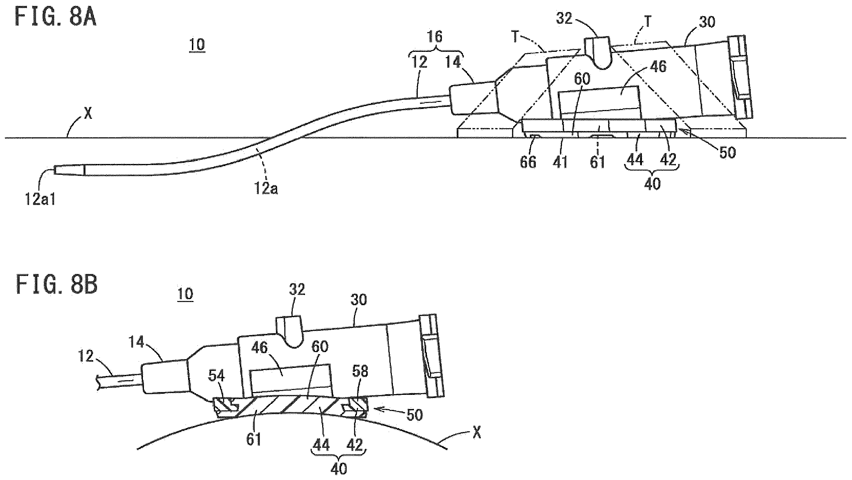

In addition, the catheter hub 14 is pasted with a medical tape T or the like when being left to indwell on the body surface X as illustrated in Fig. 8A. In the indwelling state, the lower surfaces 41 of the pair of wings 40 come into surface contact with the body surface X. Since the lower surface 41 of each of the wings 40 is inclined with respect to the axial center of the hub body 30, an excessive posture is not applied to the catheter 12 even if the soft portion 44 comes into surface contact with the body surface X. Therefore, a puncturing posture of the catheter 12 with respect to the living body is favorably maintained. In addition, the soft portion 44 can more easily follow the body surface since the frames 50 of the wings 40 are configured to be elastically deformable in the vertical direction.

Here, the soft portion 44 softer than the hard portion 42 abuts against the body surface X in the wing 40. Therefore, the wings 40 are placed while maintaining comfort of the patient. The frame 50 having the window portion 49 is elastically deformable in the vertical direction and does not strongly press an indwelling site. In addition, a part of the planar portion 60 of the soft portion 44 on the inner side of the outer peripheral portion is not supported as compared with the outer peripheral portion supported by the frame 50, and thus, even when the body surface X is curved as illustrated in Fig. 8B, the planar portion can be deformed in accordance with the curve of the body surface X.

Furthermore, the grooves 66 and 76 (see also Fig. 6) of the soft portion 44 can release sweat (moisture) generated from the body surface X, and can suppress damp during indwelling. Incidentally, a structure for suppressing the damp is not limited to the grooves 66 and 76, and may be, for example, a hole (not illustrated) penetrating through the soft portion 44 in a thickness direction. Alternatively, the soft portion 44 itself having a moisture absorbing material may be made of a material capable of absorbing the damp such as a porous body.

Furthermore, the pair of planar portions 60 (visual recognition portions 61) having transparency or translucency facilitates the visual recognition of the skin of the patient in long-term indwelling. As a result, a medical worker can satisfactorily grasp the influence of indwelling of the indwelling needle 10.

The present invention is not limited to the above-described embodiment, and various modifications can be made in accordance with a gist of the invention. For example, the hard portions 42 of the pair of wings 40 in the present embodiment are integrally molded with the hub body 30, but may be configured as separate members. For example, the root portion 46 of the hard portion 42 may have a structure (ring body or the like) that can be exteriorly provided on the outer peripheral surface of the hub body 30. Therefore, the hard portion 42 can be made of a material different from that of the hub body 30.

In addition, as in a first modification illustrated in Fig. 9A, a wing 40A may be configured to include the front frame 54 as the hard portion 42 and not to include the side frame 56 and the rear frame 58. In this case, the planar portion 60 of the soft portion 44 is provided below the front frame 54 and on the proximal side of the front frame 54. Alternatively, the soft portion 44 may be provided only below the front frame 54. Even in this case, a finger is hooked on the front frame 54 at the time of puncture, and the soft portion 44 comes into contact with the body surface X during indwelling, and thus, the same effect as that of the wing 40 can be obtained.

As in a second modification illustrated in Fig. 9B, a wing 40B may be configured to include the front frame 54 and the side frame 56 as the hard portion 42 and not to include the rear frame 58. The planar portion 60 is supported in a flatly stretched form on the hub body 30 side (the connection frame 52) and on the inner side of the front frame 54 and the side frame 56. In this manner, the wing 40B includes the front frame 54 and the side frame 56, and thus, can increase a supporting force of the planar portion 60.

As in a third modification illustrated in Fig. 9C, a wing 40C may be configured to include the front frame 54 and the rear frame 58 as the hard portion 42 and not to include the side frame 56. The planar portion 60 is supported in a flatly stretched form between the front frame 54 and the rear frame 58. In this case as well, the wing 40C can increase the supporting force of the soft portion 44.

As in a fourth modification illustrated in Fig. 9D, a wing 40D may be configured to include the soft portion 44 on the outer side of each external side (the distal end side 54a, the lateral side 56a, and the proximal end side 58a) of the frame 50 in addition to below the hard portion 42 (the frame 50). In addition, the soft portion 44 may be provided only in the vicinity of the back surface 50a of the frame 50, and the window portion 49 may penetrate therethrough. As a result, the wing 40D can more effectively achieve improvement in visibility of the body surface X, prevention of the damp, and the like while improving the operability at the time of puncture and the comfort of indwelling.

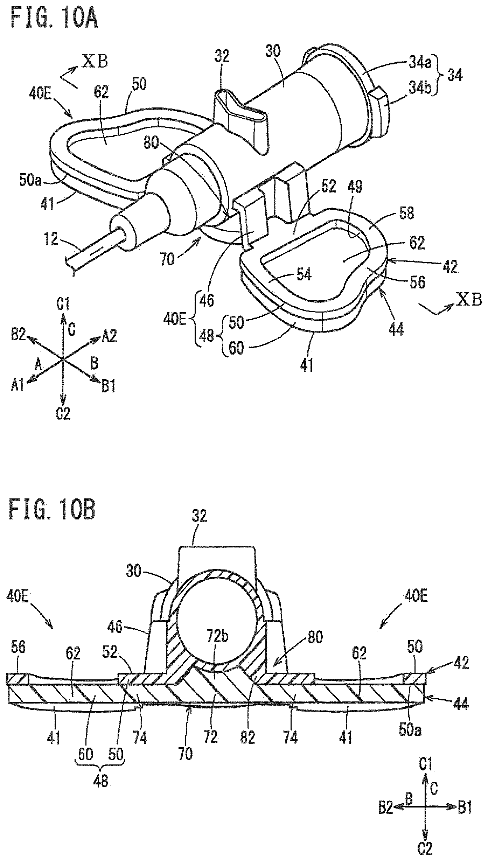

Furthermore, as in a fifth modification illustrated in Figs. 10A and 10B, a wing 40E may be configured to fix the hard portion 42 (the frame 50) and the planar portion 60 of the soft portion 44 without using the engagement structure 69 as illustrated in Fig. 6. The planar portion 60 does not include the thick portion 64 (see Fig. 5B) and is configured only by the base portion 62 having a flat upper surface, and an outer peripheral portion of the upper surface of the base portion 62 is fixed to the back surface 50a of the frame 50 by a fixing means such as adhesion or fusion. That is, the wing portion 48 of the wing 40E has a configuration in which a side surface of the frame 50 is not surrounded by the soft portion 44. Incidentally, the connecting structure 70 of the soft portion 44 may also be fixed to the support reinforcing portion 80 of the hub body 30 by adhesion, fusion, or the like.

When a thickness of the soft portion 44 in the vertical direction is set to be thicker than a thickness of the frame 50, it is possible to obtain appropriate softness that enhances the comfort of the patient. In addition, when a lower surface of the soft portion 44 (the lower surface 41 of the wings 40E) is formed in a flat shape, the soft portion 44 is easily brought into surface contact with the body surface X as a whole, and compression on the skin can be suppressed. Furthermore, the planar portion 60 configured only by the base portion 62 can have higher visibility than a structure having the thick portion 64.

Technical ideas and effects that can be grasped from the above-described embodiment are described as follows.

One aspect of the present invention is the indwelling needle 10 including the catheter 12, the catheter hub 14 fixed to the proximal end of the catheter 12, and the inner needle 18 that is detachably inserted into the catheter 12 and has the needle tip 18a protruding from the distal end of the catheter 12. The catheter hub 14 includes the hub body 30 and the wing 40 or 40A to 40E protruding from the side portion of the hub body 30. The wing 40 or 40A to 40E include the soft portion 44 softer than the catheter hub 14 and the hard portion 42 connected to the hub body 30 and harder than the soft portion 44. A part of the wing 40 or 40A to 40E that abuts on the body surface is configured by the soft portion 44. The hard portion 42 includes the front frame 54 located on the distal side of the wing 40 or 40A to 40E. The front frame 54 is connected to the hub body 30 without interposing the soft portion 44, and extends in a direction apart from the hub body 30.

According to the above, the indwelling needle 10 can enhance the comfort in the indwelling state while enhancing the operability at the time of puncture by the wing 40 or 40A to 40E including the hard portion 42 and the soft portion 44. That is, in the puncturing with the indwelling needle 10, the user can stably fix the posture of the catheter hub 14 by hooking the finger on the front frame 54 of the hard portion 42 located on the distal side of the wing 40 or 40A to 40E. As a result, the body surface X is smoothly punctured with the catheter 12 and the inner needle 18. In addition, the soft portion 44 comes into contact with the body surface X to suppress the compression on the body surface X during indwelling, and thus, a load on the patient can be reduced.

In addition, the hard portion 42 is connected to the front frame 54 and has the side frame 56 which is located on the outer side in the protruding direction of the wing 40, 40B, 40D, or 40E and extends toward the proximal side of the wing 40, 40B, 40D, or 40E, and the soft portion 44 has the planar portion 60 surrounded by the hub body 30, the front frame 54, and the side frame 56. As a result, the hard portion 42 can firmly support the soft portion 44, and the planar portion 60 can satisfactorily follow the body surface X by being curved to correspond to a bent surface or the like of the body surface X.

In addition, the hard portion 42 includes the rear frame 58 which is connected to the hub body 30, is located on the proximal side of the wing 40, 40C, 40D, or 40E, and extends in the direction apart from the hub body 30, and the soft portion 44 includes the planar portion 60 provided to extend between the front frame 54 and the rear frame 58. In this case as well, the hard portion 42 can firmly support the soft portion 44.

In addition, the hard portion 42 includes the annular frame 50 which is connected to the hub body 30 and extends over the entire circumference of the outer peripheral portion of the wing 40, 40D, or 40E, and the soft portion 44 includes the planar portion 60 surrounded by the frame 50. As a result, the outer peripheral portion of the wing 40, 40D, or 40E is significantly reinforced by the hard portion 42 in the indwelling needle 10, and the operability at the time of puncture by the user can be enhanced.

In addition, the planar portion 60 has transparency or translucency and functions as the visual recognition portion 61 that enables visual recognition of the body surface of the living body along the thickness direction of the wing 40, 40A to 40C, or 40E. As a result, the configuration in which an indwelling part on the body surface X is covered with the soft portion 44 in the wing 40, 40A to 40C, or 40E also allows the medical worker to recognize the body surface X through the planar portion 60 functioning as the visual recognition portion 61.

In addition, one or more grooves 66 are provided on the back surface of the wing 40 or 40A to 40E, and the one or more grooves 66 communicate with an outer edge of the wing 40 or 40A to 40E. As a result, the indwelling needle 10 can release sweating moisture from the body surface X through the groove 66, and can suppress damp of the indwelling site.

In addition, the soft portion 44 and the hard portion 42 engage with each other to form the engagement structure 69. As a result, the indwelling needle 10 can reliably support the soft portion 44 by the hard portion 42.

In addition, a pair of the wings 40 or 40A to 40E is provided in the hub body 30 and protrudes in directions opposite to each other, and the soft portion 44 is formed to be continuous over the pair of wings 40 or 40A to 40E and is attached to each of the hard portions 42 constituting the pair of wings 40 or 40A to 40E. As a result, the number of components of the indwelling needle 10 can be reduced, and manufacturing cost can be reduced.

In addition, the lower surface of the wing 40 or 40A to 40E is inclined in the direction facing the axial center of the hub body 30 toward the distal side of the hub body 30. As a result, the indwelling needle 10 can bring the distal end of the hub body 30 close to the body surface X by inclining the axial center of the hub body 30 in the indwelling of the wing 40 or 40A to 40E, and an insertion posture of the catheter 12 in the body surface X is stabilized.

In addition, the wing 40 or 40A to 40E is configured to be elastically deformable in a direction orthogonal to the axial direction of the hub body 30 and in a direction orthogonal to the extending direction of the front frame 54. As a result, when the wing 40 or 40A to 40E is placed on the body surface, the soft portion 44 more easily follows the body surface.

## Claims

1. An indwelling needle comprising:
a catheter;
a catheter hub fixed to a proximal end of the catheter; and
an inner needle that is detachably inserted into the catheter and has a needle tip protruding from a distal end of the catheter, wherein
the catheter hub includes a hub body and a wing protruding from a side portion of the hub body,
the wing includes a soft portion softer than the catheter hub and a hard portion connected to the hub body and harder than the soft portion,
a part of the wing that abuts on a body surface is configured by the soft portion,
the hard portion includes a front frame located on a distal side of the wing, and
the front frame is connected to the hub body without interposing the soft portion, and extends in a direction apart from the hub body.

2. The indwelling needle according to claim 1, wherein
the hard portion includes a side frame which is connected to the front frame, is located on an outer side in a protruding direction of the wing, and extends toward a proximal side of the wing, and
the soft portion includes a planar portion surrounded by the hub body, the front frame, and the side frame.

3. The indwelling needle according to claim 1, wherein
the hard portion includes a rear frame which is connected to the hub body, is located on a proximal side of the wing, and extends in a direction away from the hub body, and
the soft portion includes a planar portion provided to extend between the front frame and the rear frame.

4. The indwelling needle according to claim 1, wherein
the hard portion includes an annular frame which is connected to the hub body and extends around an entire circumference of an outer peripheral portion of the wing, and
the soft portion includes a planar portion surrounded by the frame.

5. The indwelling needle according to any one of claims 2 to 4, wherein
the planar portion has transparency or translucency, and functions as a visual recognition portion which enables visual recognition of a body surface of a living body along a thickness direction of the wing.

6. The indwelling needle according to any one of claims 2 to 5, wherein
one or more grooves are provided on a back surface of the wing, and
the one or more grooves communicate with an outer edge of the wing.

7. The indwelling needle according to any one of claims 1 to 6, wherein
the soft portion and the hard portion engage with each other to form an engagement structure.

8. The indwelling needle according to any one of claims 1 to 7, wherein
a pair of the wings is provided in the hub body and protrudes in directions opposite to each other, and
the soft portion is formed to be continuous over the pair of wings, and is attached to each of the hard portions constituting the pair of wings.

9. The indwelling needle according to any one of claims 1 to 8, wherein
a lower surface of the wing is inclined in a direction facing an axial center of the hub body toward a distal side of the hub body.

10. The indwelling needle according to any one of claims 1 to 9, wherein
the wing is configured to be elastically deformable in a direction orthogonal to an axial direction of the hub body and in a direction orthogonal to an extending direction of the front frame.
